# EUROPÄISCHE PATENTSCHRIFT

(11) **EP 2 301 424 B1**
(45) Veröffentlichungstag und Bekanntmachung des Hinweises auf die Patenterteilung: **08.07.2015**
(21) Anmeldenummer: 09075449.0
(22) Anmeldetag: 29.09.2009
(51) Int. Cl.: A61B 3/12, A61B 3/13, A61B 3/14, A61F 9/008

(54) **Ophthalmoskop mit einer Laservorrichtung**
Ophthalmoscope with a laser device
Ophtalmoscope doté d'un dispositif laser

(43) Veröffentlichungstag der Anmeldung: 30.03.2011
(73) Patentinhaber: OD-OS GmbH, 14513 Teltow (DE)
(72) Erfinder: Liesfeld, Ben, 14469 Potsdam (DE); Teiwes, Winfried, 14532 Kleinmachnow (DE); Weber, Ulrike, 14480 Potsdam (DE); Amthor, Kay-Uwe, 14469 Potsdam (DE); Kirsch, Stephan, 16547 Birkenwerder (DE)
(74) Vertreter: Pfenning, Meinig & Partner GbR

(56) Entgegenhaltungen:
- EP-A- 0 791 361
- WO-A-01/95791
- RICHARD S KAISER ET AL: "Laser Burn Intensity and the Risk for Choroidal Neovascularization in the CNVPT Fellow Eye Study" OPHTALMOLOGIE, PARIS, FR, Bd. 119, 1. Juni 2001 (2001-06-01), Seiten 826-832, XP007911267
- YANNUZZI L A ET AL: "Ophthalmic fundus imaging: today and beyond" AMERICAN JOURNAL OF OPHTHALMOLOGY, OPHTHALMIC PUBL, CHICAGO, IL, US, Bd. 137, Nr. 3, 1. März 2004 (2004-03-01), Seiten 511-524, XP004755596 ISSN: 0002-9394

## Beschreibung

Die vorliegende Erfindung betrifft ein Ophthalmoskop mit einer Laservorrichtung zur Laserbestrahlung eines Auges und einer Kamera zum Erfassen eines Bildes des Auges

Ophthalmoskope mit Laservorrichtungen werden vielfach zur Beobachtung und zur Behandlung von Augen eingesetzt. Eine wichtiges Behandlungsverfahren stellt dabei die Photokoagulation dar, bei der ein Hintergrund des Auges, auch als Fundus bezeichnet, in einem Behandlungsgebiet koaguliert oder verödet wird. Für eine erfolgreiche Durchführung einer solchen Behandlung ist es notwendig einen Therapiestrahl exakt auf dem Behandlungsgebiet zu positionieren. Außerdem muss eine Auswirkung einer Laserbehandlung auf ein behandeltes Gewebe im Auge kontrolliert und verifiziert werden.

Zu diesem Zweck wird üblicherweise das zu behandelnde Auge während einer Lasertherapie mit sichtbarem Licht beleuchtet. Dies hat den Vorteil, dass unter sichtbarem (weißem) Licht eine physiologische Wirkung der Laserbestrahlung, welche sich in der Regel in Form einer Bleichung des bestrahlten Gewebes manifestiert, besonders deutlich sichtbar ist. Insbesondere wird daher auch bereits vor einer Laserbehandlung eine versuchsweise Laserbestrahlung des Auges an unkritischen Regionen des Auges vorgenommen, um unter Weißlichtbeleuchtung eine Bestimmung einer optimalen Bestrahlungsdauer, Bestrahlungsintensität und weiterer Parameter durchzuführen Derartige Beleuchtungssysteme, welche z.T. auch mit einer Kamera zum Erfassen eines Bildes des Auges ausgestattet sind, gemäß dem Oberbegriff des Anspruchs 1 sind z.B. aus folgenden Dokumenten bekannt: · WO 01/95791 · RICHARD S KAISER ET AL, Laser Burn Intensity and the Risk for Choroidal Neovascularization in the CNVPT Fellow Eye Study, OPHTALMOLOGIE, PARIS, FR, Bd. 119, 826-832 YANNUZZI L A ET AL, Ophthalmic fundus imaging: today and beyond, AMERICAN JOURNAL OF OPHTHALMOLOGY, OPHTHALMIC PUBL, CHICAGO, IL, US, Bd. 137, Nr. 3, 511-524, EP0791361.

Eine Beleuchtung des Auges mit sichtbarem Licht wird aber von den meisten Patienten als blendend und äußerst unangenehm empfunden, so dass die betreffenden Patienten oft mit unwillkürlichen Augenbewegungen auf die Beleuchtung reagieren. Diese Augenbewegungen erschweren die Beobachtung sowie die Erfassung eines Bildes des Auges beträchtlich. Besonders nachteilig wirken sich Augenbewegungen auf eine Genauigkeit aus, mit der eine Laserbestrahlung auf einen gewünschten Behandlungsbereich appliziert werden kann und verursachen so ein beachtliches Risiko einer unbeabsichtigten Bestrahlung eines sensitiven Bereichs des Auges. Außerdem machen Augenbewegungen in der Regel eine neue Positionierung eines Laserstrahls notwendig, was eine erhebliche Verlängerung einer Behandlungsdauer zur Folge haben kann, insbesondere falls eine große Anzahl von Bereichen des Auges einzeln bestrahlt werden müssen.

Es ist daher üblich, ein Kontaktglas auf die Kornea des Auges aufzusetzen und auf diese Weise die genannten Augenbewegungen mechanisch zu unterdrücken. Dies führt jedoch zu Reizungen des Auges und ist in der Regel nur unter lokaler Betäubung durchführbar.

Der vorliegenden Erfindung liegt also die Aufgabe zugrunde ein Ophthalmoskop vorzuschlagen, welches die genannten Probleme löst oder zumindest abmildert, welches also zu einer Reduzierung unwillkürlicher Augenbewegungen während einer Beobachtung des Auges und insbesondere während einer Laserbehandlung des Auges, geeignet ist. Ferner ist ein entsprechendes Verfahren zum Beobachten eines des Auges, insbesondere zur Kontrolle einer therapeutischen Behandlung eines Bereichs des Auges, und schließlich ein Verfahren zur Durchführung einer Laserbehandlung vorzuschlagen, welches das Problem unwillkürlicher Augenbewegungen löst oder zumindest abmildert.

Diese Aufgabe wird erfindungsgemäß gelöst durch ein Ophthalmoskop gemäss dem unabhängigen Anspruch 1.

Weiterentwicklungen der Erfindung sind Gegenstände der Unteransprüche.

Demnach umfasst ein erfindungsgemäßes Ophthalmoskop mit einer Laservorrichtung zur Laserbestrahlung eines Auges, insbesondere zum Durchführen einer Photokoagulation auf einem Hintergrund des Auges, eine Beleuchtungseinheit zum Beleuchten des Auges sowie eine Kamera zum Erfassen eines Bildes des Auges, wobei die Beleuchtungseinheit eingerichtet ist zum Erzeugen von sichtbarem und infrarotem Licht, wobei das Ophthalmoskop ferner eine Steuereinheit umfasst, welche dazu eingerichtet ist die Beleuchtungseinheit zum Erzeugen eines Lichtimpulses von sichtbarem Licht anzusteuern und ein während des Lichtimpulses von der Kamera erfasstes Kontrollbild des Auges aus der Kamera auszulesen.

Das erfindungsgemäße Ophthalmoskop mit der Laservorrichtung hat die Eignung, ein Kontrollbild des Auges, insbesondere eines Hintergrundes des Auges, während einer Beleuchtung des Auges mit einem Lichtimpuls von sichtbarem Licht zu erfassen. Durch eine vorzugsweise möglichst kurze Dauer des Lichtimpulses führt der Lichtimpuls zu keiner oder nur zu einer geringfügigen Blendung des Auges und zu keinem oder einem nur geringen Zusammenziehen einer Pupille des Auges während einer Untersuchung oder Laserbehandlung des Auges.

Vorzugsweise besitzt der Lichtimpuls ein breites Frequenzspektrum, um ein möglichst detailreiches, farbiges Kontrollbild zu ermöglichen. Alternativ kann aber auch vorgesehen sein, dass der Lichtimpuls nur einen vorgegebenen schmalen Ausschnitt des sichtbaren Frequenzspektrums umfasst, um eine spezifische Schichttiefe eines Gewebes des Auges zu untersuchen. Auf diese Weise lässt sich insbesondere eine physiologische Wirkung einer Laserbestrahlung, etwa eines Augenhintergrundes, innerhalb unterschiedlicher Schichttiefen je nach Wellenlängen des Lichtimpulses getrennt analysieren. Zu diesem Zweck weist die Beleuchtungsvorrichtung vorzugsweise eine oder mehrere Lichtquellen auf, welche jeweils zur Erzeugung von Licht innerhalb eines schmalen Frequenzbereichs geeignet ist, wie etwa Leuchtdioden.

In einer alternativen Ausführungsform ist die Steuereinheit dazu eingerichtet die Beleuchtungseinheit zum Erzeugen eines Lichtimpulses innerhalb eines infraroten Frequenzbereichs anzusteuern und ein während des Lichtimpulses von der Kamera erfasstes Kontrollbild des Auges aus der Kamera auszulesen. Damit hat diese Ausführungsform die Eignung, Strukturen in einer größeren Tiefe innerhalb des Gewebes des Auges abzubilden als dies mit sichtbarem Licht möglich ist. Generell lassen sich Gewebestrukturen mit Licht etwa innerhalb einer Eindringtiefe des Lichts in das Gewebe abbilden. Da die Eindringtiefe des Lichts mit der Wellenlänge des Lichts skaliert, besitzt insbesondere infrarotes Licht eine größere Eindringtiefe als sichtbares Licht.

Vorzugsweise ist ferner die Kamera eine möglichst hochauflösende Farbbildkamera, welche beispielsweise mit einem CCD-Sensor ausgestattet ist. In einer weiteren Ausführungsform weist das Ophthalmoskop eine Beobachtungsvorrichtung auf, vorzugsweise mit einem Bildschirm, welche mit der Kamera verbunden ist, zum Beobachten der von dem Ophthalmoskop erfassten Bilder des Auges.

Besonders vorteilhaft ist die Kamera außer für sichtbares Licht auch für infrarotes Licht sensitiv. Da infrarotes Licht von einem menschlichen Auge nicht wahrgenommen wird, kann das Auge während der Untersuchung kontinuierlich mit infrarotem Licht beleuchtet werden, ohne damit eine Blendung des Patienten zu bewirken. Auf diese Weise lässt sich mit dem Ophthalmoskop eine kontinuierliche Abfolge von Einzelbildern des Auges während der Untersuchung oder der Behandlung des Auges erzeugen, welche im Folgenden auch als Echtzeitbild bezeichnet wird. Obwohl unter infraroter Beleuchtung manche Details des Augenhintergrunds nicht erkennbar sind, wie beispielsweise Läsionen der Netzhaut aufgrund der Laserbestrahlung, erlaubt das unter infraroter Beleuchtung erzeugte Echtzeitbild aber eine kontinuierliche Beobachtung des Auges ohne eine nachteilige Blendwirkung und somit insbesondere eine sichere Positionierung eines Ziellaserstrahls auf einen zu behandelnden Bereich im Augenhintergrund.

In einer Weiterentwicklung des Ophthalmoskops ist vorgesehen, dass die Steuereinheit eingerichtet ist nach einer vorgegebenen Wartezeitspanne nach einer Laserbestrahlung des Auges die Beleuchtungseinheit zum Erzeugen des Lichtimpulses anzusteuern und das während des Lichtimpulses von der Kamera erfasste Kontrollbild aus der Kamera auszulesen. Dabei ist die Wartezeitspanne vorzugsweise so lang gewählt, dass durch die Laserbestrahlung induzierte physiologische Prozesse, wie beispielsweise eine Denaturierung von Proteinen innerhalb des bestrahlten Gewebes, weitgehend abgeschlossen sind, so dass eine möglichst sichere Bewertung eines Erfolges der Bestrahlung anhand des Kontrollbildes durchführbar ist. Die Wartezeitspanne liegt vorzugsweise in einem Bereich zwischen 1 Millisekunde und 5 Sekunden, besonders bevorzugt in einem Bereich zwischen 40 Millisekunden und 1 Sekunde.

In einer alternativen Ausführungsform der Erfindung ist die Steuereinheit dazu eingerichtet nach einer vorgegebenen Wartezeitspanne nach Abschluss einer Serie einer vorgegebenen Anzahl einzelner Laserbestrahlungen die Beleuchtungseinheit zum Erzeugen des Lichtimpulses anzusteuern und das während des Lichtimpulses von der Kamera erfasste Kontrollbild aus der Kamera auszulesen. Auf dieser Weise ist eine zügige Durchführung einer Laserbehandlung erreichbar, insbesondere dann, wenn eine große Anzahl einzelner Laserbestrahlungen (Laserimpulse) appliziert werden muss. Die letztgenannte Wartezeitspanne liegt wiederum vorzugsweise innerhalb der oben angegebenen Bereiche.

In einer Weiterentwicklung des erfindungsgemäßen Ophthalmoskops ist vorgesehen, dass die Steuereinheit eingerichtet ist eine vorgegebene Zeitspanne vor einer Laserbestrahlung die Beleuchtungseinheit zum Erzeugen des Lichtimpulses anzusteuern und das während des Lichtimpulses von der Kamera erfasste Kontrollbild als ein Referenzbild aus der Kamera auszulesen. Ein solches Referenzbild hat den Vorteil, dass es sich besonders gut für einen Vergleich mit einem unmittelbar nach der betreffenden Laserbestrahlung während des Lichtimpulses von der Kamera erfassten Kontrollbild eignet, weil beide Kontrollbilder innerhalb eines vorzugsweise möglichst kurzen Zeitintervalls erzeugt wurden (welches sich als Summe der genannten Zeitspanne, einer Laserimpulsdauer und der oben definierten Wartezeitspanne ergibt) und das Auge innerhalb dieses Zeitintervalls wahrscheinlich nicht oder nur wenig bewegt hat. Dies erlaubt eine besonders gute Bildregistration (siehe unten) des Referenzbildes (vor der Laserbehandlung) mit dem Kontrollbild (nach der Laserbehandlung). Die genannte Zeitspanne liegt vorzugsweise in einem Bereich zwischen 5 s und 40 ms, besonders bevorzugt in einem Bereich zwischen 200 ms und 40 ms.

In einer Weiterentwicklung der Erfindung ist vorgesehen, dass die Steuereinheit mit einer Eingabeschnittstelle zum Eingeben eines Auslösesignals verbunden ist, wobei die Steuereinheit dazu eingerichtet ist nach Eingabe des Auslösesignals die Beleuchtungseinheit zum Erzeugen des Lichtimpulses anzusteuern und das während des Lichtimpulses von der Kamera erfasste Kontrollbild aus der Kamera auszulesen. Auf diese Weise hat ein behandelnder Arzt jederzeit während einer Untersuchung oder einer Behandlung die Möglichkeit, eine Kontrollbild, vorzugsweise in Form einer Farbbildaufnahme, zu erzeugen, ohne dass der Patient dabei unnötig stark geblendet wird.

In einer Weiterentwicklung des erfindungsgemäßen Ophthalmoskops ist vorgesehen, dass sie eine mit der Kamera verbundene digitale Bildverarbeitungseinheit beinhaltet, die dazu eingerichtet ist aus einem Paar von Kontrollbildern ein Differenzwertbild zu erzeugen. Zu diesem Zweck ist die Bildverarbeitungseinheit mit einem Speicher zum Speichern der Kontrollbilder ausgestattet und ferner dazu eingerichtet, eine vorzugsweise merkmalsbasierte Bildregistrierung dieser beiden Kontrollbilder durchzuführen, so dass eventuelle Augenbewegungen in einer Zwischenzeit zwischen der Aufnahme der beiden Kontrollbilder eliminiert werden können. In einer vereinfachten Ausführungsform ist die Bildverarbeitungseinheit dazu eingerichtet, anstelle einer solchen Bildregistrierung nur solche Bildpunkte der beiden Kontrollbilder für die Erzeugung des Differenzwertbild zu verwendet werden, zwischen denen keine Augenbewegung stattgefunden hat, welche also kongruent zueinander sind.

Das Differenzwertbild besteht aus Pixelwerten, welche jeweils einen Differenzwert abbilden, der jeweils durch eine Differenz zwischen zugehörigen Pixelwerten der beiden (bildregistrierten) Kontrollbilder definiert ist. Ein solches Differenzwertbild hat den Vorteil, dass auf ihm Unterschiede zwischen den beiden Kontrollbildern besonders gut erkennbar und quantifizierbar sind. Daher handelt es sich vorzugsweise bei dem ersten der beiden Kontrollbilder um ein oben beschriebenes Referenzbild, welches vor einer Laserbestrahlung aufgenommen und in dem Speicher zwischengespeichert worden ist, und bei dem zweiten Referenzbild um ein Kontrollbild, welches nach dieser Laserbestrahlung aufgenommen worden ist. Anhand eines solchen Differenzwertbildes lässt sich die physiologische Wirkung und damit der therapeutische Erfolg dieser einzelnen Laserbestrahlungen des Auges besonders sicher beurteilen und quantifizieren lässt.

In einer Weiterentwicklung ist vorgesehen, dass die Bildverarbeitungseinheit dazu eingerichtet ist, das Differenzwertbild gemäß vorgegebener Wertebereiche für Differenzwerte des Differenzwertbildes zu segmentieren, um eine besonders übersichtliche und leicht zu interpretierende Darstellung des Differenzwertbildes zu erzielen. Vorzugsweise ist die Bildverarbeitungseinheit dazu eingerichtet das Differenzwertbild in Bildsegmente einer ersten, einer zweiten und einer dritten Segmentklasse zu segmentieren, wobei ein Bildsegment der ersten Segmentklasse einen Behandlungserfolg innerhalb dieses Bildsegments, ein Bildsegment der zweiten Segmentklasse eine notwendige Behandlungswiederholung innerhalb dieses Bildsegments und ein Bildsegment der dritten Segmentklasse eine notwendige Reduktion einer Laserintensität innerhalb dieses Bildsegments signalisiert. Auf diese Weise ist es einem behandelnden Arzt möglich auf einen Blick zu entscheiden, ob eine Laserbestrahlung, welche zwischen den beiden zum Differenzwertbild gehörigen Kontrollbilder appliziert wurde, den gewünschten Erfolg hatte, ob sie möglicherweise wiederholt werden sollte oder ob die Laserintensität für den betreffenden Bereich angepasst werden muss.

In einer Weiterentwicklung der Erfindung ist vorgesehen, dass die Bildverarbeitungseinheit dazu eingerichtet ist einen Bildausschnitt eines Kontrollbildes auszuwählen, zu vergrößern und das Kontrollbild teilweise mit dem ausgewählten und vergrößerten Bildausschnitt zu überlagern, wobei der ausgewählte und vergrößerte Bildausschnitt eines mit einer Laserbestrahlung behandelten Bereichs des Auges beinhaltet. Der ausgewählte, vergrößerte und in das Kontrollbild überlagernd eingefügte Bildausschnitt dient einer besonders deutlichen Darstellung von Details des behandelten Bereichs des Auges und zur besseren Beurteilung eines physiologischen Effektes der Laserbestrahlung des Bereichs. In einer alternativen Ausführungsform der Erfindung ist die Steuereinheit eingerichtet für den ausgewählten Bildausschnitt ein Differenzwertbild, vorzugsweise mit einem entsprechenden Bildausschnitt eines zweiten unmittelbar vor der Laserbestrahlung während eines Lichtimpulses von der Kamera erfassten Kontrollbildes wie oben beschrieben zu erzeugen, anschließend dieses Differenzwertbild zu vergrößern und das Kontrollbild mit diesem Differenzwertbild teilweise zu überlagern.

In einer Weiterentwicklung des erfindungsgemäßen Ophthalmoskops ist vorgesehen, dass die Laservorrichtung eine Strahllenkungseinheit beinhaltet zur Lenkung eines von der Laservorrichtung erzeugten Laserstrahls. Ein solches Ophthalmoskop hat die Eignung den Laserstrahl auf einem gewünschten Bereich des Auges zu positionieren. Zu diesem Zweck ist die Laservorrichtung vorzugsweise zur Erzeugung eines Ziellaserstrahls eingerichtet mit einer reduzierten Intensität, welcher keine oder nur unerhebliche physiologische Wirkungen auf das Gewebe des Auges besitzt. Vorzugsweise ist diese Strahllenkungseinheit dazu eingerichtet, den Laserstrahl während des Lichtimpulses von sichtbarem Licht aus einem Beobachtungsbereich der Kamera (6) heraus zu lenken. Auf diese Weise wird insbesondere erreicht, dass der Ziellaser während der Erfassung eines Kontrollbildes vor oder nach einer Laserbestrahlung eines Behandlungsbereichs diesen nicht mehr bestrahlt und somit auch nicht mehr Bildinformationen des Behandlungsbereiches überstrahlt. Ein solches Herauslenken und vorzugsweise anschließendes Hineinlenken des Ziellaserstrahls hat den Vorteil, dass es innerhalb einer sehr kurzen Zeitspanne durchführbar ist, wohingegen ein vollständigen Deaktivieren und nachfolgendes Reaktivieren des Lasers eine dazwischen liegende Pause erfordert.

In einer Weiterentwicklung weist das Ophthalmoskop in einem für das (zu untersuchende oder zu behandelnde) Auge sichtbaren Bereich eine Anzeigevorrichtung auf zur Darstellung eines Fixationspunkts zum Fixieren des Auges auf den Fixationspunkt, wodurch unwillkürlichen Augenbewegungen zusätzlich entgegengewirkt werden kann.

Mit einem Ophthalmoskop hier vorgeschlagener Art kann ein Verfahren zum Erfassen eines Kontrollbilds eines Auges durchgeführt werden , wobei eine Beleuchtung des Auges mit sichtbarem Licht möglichst kurzzeitig zur gleichzeitigen Aufnahme eines Kontrollbildes mit der Kamera durchgeführt wird, wodurch sich unwillkürliche Augenbewegungen des Patienten aufgrund einer Blendung des Patienten weitgehend vermeiden lassen. Vorzugsweise wird eine kontinuierliche Beobachtung des Auges unter einer Infrarotlichtbeleuchtung des Auges durchgeführt, wobei Bildsignale der Kamera an eine Beobachtungsvorrichtung, beispielsweise mit einem Bildschirm, weitergeleitet werden. Ferner sieht das erfindungsgemäße Verfahren zur Laserbehandlung eines Auges mit einem Ophthalmoskop, insbesondere zur Durchführung einer Photokoagulation eines Hintergrundes des Auges, vor, dass mit einer Laservorrichtung eines Ophthalmoskops eine Laserbestrahlung des Auges durchgeführt wird und nach einer vorgegebenen Wartezeitspanne nach der Laserbestrahlung das Auge mit einer Beleuchtungsvorrichtung des Ophthalmoskops mit einem Lichtimpuls von sichtbarem Licht beleuchtet wird und gleichzeitig mit einer Kamera des Ophthalmoskops ein Kontrollbild des Auges erfasst wird. Dabei wird das Auge während der Behandlung vorzugsweise kontinuierlich unter infraroter Beleuchtung beobachtet. Ferner werden diese Verfahren vorzugsweise mit einem Ophthalmoskop hier vorgeschlagener Art durchgeführt.

Im Folgenden werden spezielle Ausführungsformen der Erfindung anhand von Figuren 1 bis 5 näher beschrieben. Es zeigt:
- Fig. 1: eine schematische Darstellung der beispielhaften Ausführungsform des hier vorgeschlagenen Ophthalmoskops,
- Fig. 2: eine zeitliche Abfolge von Bildern eines Augenhintergrundes,
- Fig. 3: eine schematische Darstellung eines Referenzbildes,
- Fig. 4: eine schematische Darstellung eines Referenzbildes mit einem zusätzlich eingeblendeten Bildausschnitt und
- Fig. 5: eine schematische Darstellung eines Differenzenbildes.

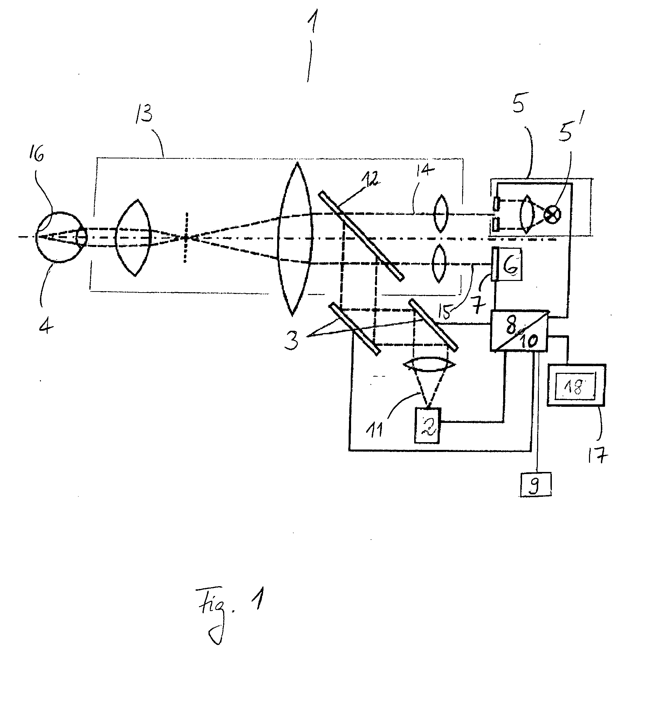

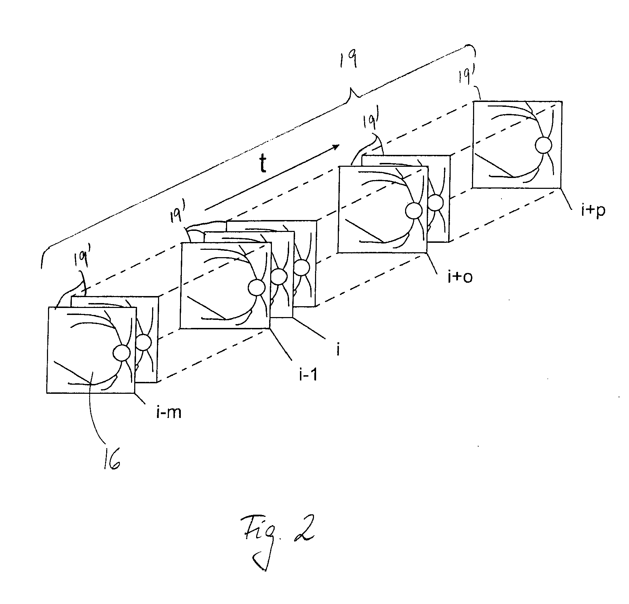

Figur 1 zeigt eine schematische Darstellung einer speziellen Ausführungsform eines Ophthalmoskops 1 hier vorgeschlagener Art. Es umfasst eine Laservorrichtung 2 mit einer Strahllenkungseinheit 3 zur Laserbestrahlung eines Auges 4. Das Ophthalmoskop umfasst ferner eine Beleuchtungseinheit 5 zur Beleuchtung des Auges 4 mit wahlweise infrarotem und/oder sichtbarem Licht mittels Leuchtdioden 5', eine Kamera 6 mit einem CCD-Sensor 7, welcher sowohl für infrarotes wie sichtbares Licht empfindlich ist, und eine mit der Kamera 6 verbundene Steuereinheit 8. Die Steuereinheit 8 ist dazu eingerichtet, nach einer Wartezeitspanne von 200 ms nach einer Laserbestrahlung des Auges 4 mit einem Therapielaserstrahl die Beleuchtungseinheit 5 zum Erzeugen eines Lichtimpulses von sichtbarem Licht anzusteuern und ein während des Lichtimpulses von der Kamera 6 erfasstes Kontrollbild des Auges 4, vgl. Figuren 2 bis 5, aus der Kamera 6 auszulesen.

Die Steuereinheit 8 ist ferner dazu eingerichtet, auch vor einer Laserbestrahlung des Auges 4 mit einem Therapielaserstrahl die Beleuchtungseinheit 5 zum Erzeugen des Lichtimpulses anzusteuern und ein weiteres Kontrollbild als ein Referenzbild aus der Kamera 6 auszulesen. Zusätzlich ist die Steuereinheit 8 mit einer Eingabeschnittstelle 9 zum Eingeben eines Auslösesignals verbunden, wobei die Steuereinheit 8 dazu eingerichtet ist nach Eingabe des Auslösesignals die Beleuchtungseinheit 5 zum Erzeugen des Lichtimpulses anzusteuern und das während des Lichtimpulses von der Kamera 6 erfasste Kontrollbild aus der Kamera 6 auszulesen.

In die Steuereinheit 8 ist eine Bildverarbeitungseinheit 10 integriert, welche über die Steuereinheit mit der Kamera 6 verbunden ist und zur Verarbeitung von Bildsignalen der Kamera 6 in Echtzeit eingerichtet ist.

Die Bildverarbeitungseinheit 10 ist außerdem dazu eingerichtet ist einen Bildausschnitt eines Kontrollbildes auszuwählen, zu vergrößern und das Kontrollbild teilweise mit dem ausgewählten und vergrößerten Bildausschnitt zu überlagern, wobei der ausgewählte und vergrößerte Bildausschnitt eines mit einer Laserbestrahlung behandelten Bereichs des Auges beinhaltet. Ferner ist die Bildverarbeitungseinheit 10 dazu eingerichtet aus einem Paar von Kontrollbildern ein Differenzwertbild zu erzeugen. Zu diesem Zweck ist die Bildverarbeitungseinheit 10 mit einem Speicher zum Speichern der Kontrollbilder ausgestattet und ferner dazu eingerichtet, eine merkmalsbasierte Bildregistrierung dieser beiden Kontrollbilder durchzuführen.

Die Strahllenkungseinheit 3 ist insbesondere dazu eingerichtet einen Laserstrahl 11 der Laservorrichtung 2, welcher wahlweise durch einen Ziellaserstrahl, den Therapielaserstrahl oder eine Überlagerung von beiden gegeben ist, während des Lichtimpulses von sichtbarem Lichts aus einem von der Kamera 6 erfassten Beobachtungsbereich heraus zu lenken. Ein Strahlteiler 12 dient der Einkopplung des Laserstrahls 11 in ein optisches System 13, welches einer Abbildung eines aus dem Beleuchtungssystem 5 stammenden Beleuchtungsstrahls 14 und des dem Beleuchtungsstrahl überlagerten Ziel- oder Therapielaserstrahls 11 auf das Auge 4, im vorliegenden Fall auf einen Hintergrund 16 des Auges 4, dient. Außerdem dient das optische System 13 gleichzeitig zur Abbildung eines Beobachtungsstrahls 15, welcher durch die Reflexion des Beleuchtungsstrahls 14 (und gegebenenfalls des Laserstrahls 11, falls dieser nicht wie oben beschrieben durch die Strahllenkungseinheit abgelenkt ist) am Auge 4 gegeben ist, auf den Sensor 7 der Kamera 6.

Eine Beobachtungsvorrichtung 17 mit einem Bildschirm 18 ist mit der Steuereinheit 8 und der in der Steuereinheit 8 integrierten Bildverarbeitungseinheit 10 verbunden zur optischen Darstellung der von der Kamera 6 erfassten Bilder des Augenhintergrundes 16.

Anhand der Nachfolgenden Figuren 2 bis 5 wird ein Verfahren zum Erfassen eines Kontrollbildes des Augenhintergrundes 16 beschrieben, welches mit dem hier beschriebenen beispielhaften Ausführungsbeispiel 1 der Erfindung durchgeführt wird. Ferner wird ein Verfahren zur Durchführung einer Photokoagulation beschrieben, welches ebenfalls mit dem Ausführungsbeispiel 1 an dem Augenhintergrund 16 durchgeführt wird. Die Nummerierung der Merkmale des Ausführungsbeispiels 1 wird dabei beibehalten.

Figur 2 zeigt eine schematische Darstellung einer zeitlichen Abfolge 19 von Bildern 19', welche durch die anhand Figur 1 beschriebene Ausführungsform aufgenommen wurden. Der mit dem Buchstaben t bezeichnete Pfeil sowie die Indizierungen i-m, i-1, i, i+o und i+p geben dabei die Richtung und die Reihenfolge der zeitlichen Abfolge 19 der Bilder 19' an, wobei die Indizes i, m, o und p positive, ganze Zahlen sind. Ferner gilt, dass p größer oder gleich o ist.

Zur Erzeugung dieser Bildfolge 19 wird das Auge 4 mit der Beleuchtungseinheit 5 kontinuierlich mit einem infraroten, unsichtbaren Beleuchtungsstrahl 14 bestrahlt. Mit dem optischen System 13 wird dieser Beleuchtungsstrahl 14 auf den Hintergrund 16 des Auges 4 abgebildet und dessen Reflexion am Augenhintergrund 16, welcher durch den Beobachtungsstrahl 15 gegeben ist, auf den Sensor 7 der Kamera 6 abgebildet. Der Sensor 7 erzeugt eine zeitliche Abfolge von Bildsignalen, welche von der Kamera 6 an die in der Steuereinheit 8 integrierte Bildverarbeitungseinheit 10 weitergeleitet werden. Von der Bildverarbeitungseinheit werden diese Bildsignale schließlich an die Beobachtungsvorrichtung 17 übertragen, welche die Bildsignale auf dem Bildschirm 18 als zeitliche Bildfolge 19 (als Echtzeitbild) abbildet. Eine Bildaufnahmerate der Kamera 6 beträgt im vorliegenden Beispiel etwa 25 Hz, d.h. eine zugehörige Belichtungszeit der einzelnen Bilder 19' beträgt etwa 40 ms.

In einem Verfahren zur Erzeugung eines Kontrollbildes des Auges 4 aktiviert die Steuereinheit 9 die Beleuchtungseinheit zum Erzeugen eins Lichtimpulses, welcher möglichst kurzzeitig ausgestaltet ist, um eine Blendung des Auges zu vermeiden. Es ist daher prinzipiell ausreichend die Dauer des Lichtimpulses auf die Beleuchtungszeit der einzelnen Bilder 19' der Bildfolge 19 zu begrenzen, da ein einziges der Bilder 19' als Kontrollbild ausreichend ist. Der Lichtimpuls wird beispielsweise während der Belichtungszeit des Bildes 19' mit dem Index i erzeugt. Dieses Bild 19' mit dem Index i wird nun aus der Kamera ausgelesen als Kontrollbild des Auges 4. Anschließend wird das Kontrollbild von der Bildverarbeitungseinheit 10 gegebenenfalls gespeichert und/oder weiterverarbeitet, an die Beobachtungsvorrichtung 17 weitergeleitet und durch den Bildschirm 18 abgebildet. Ein solches Verfahren kann durch einen Bediener beispielsweise durch Betätigung der Eingabeschnittstelle 9 ausgelöst werden.

Eine therapeutische Laserbestrahlung des Auges 4 mit dem Therapielaserstrahl 3 hat üblicherweise eine Dauer (Impulsdauer) in einem Bereich zwischen 10 ms und 1000 ms und kann somit länger als die Belichtungsdauer der einzelnen Bilder 19' sein. Im folgenden beispielhaften Verfahren zur Durchführung einer Laserbehandlung des Auges 4 wird während einer Belichtungszeit des Bildes 19' mit Index i eine Bestrahlung des Augenhintergrund 16 mit einem Therapielaserstrahl mit einer Impulsdauer von 400 ms begonnen. Der Index i+o markiert das erste Bild, welches nach Beendigung der Bestrahlung aufgenommen wird. In diesem Beispiel hat o demnach den Wert 11, da während der Belichtungszeit des Bildes mit Index i+10 = i die Bestrahlung beendet wird. Wie oben beschrieben, ist die Steuereinheit 8, oder im vorliegenden Beispiel die in ihr integrierte Bildverarbeitungseinheit 10, dazu eingerichtet, nach Ablauf einer vorgegebenen Wartezeitspanne nach einer Laserbestrahlung ein Kontrollbild aus der Kamera auszulesen. Im vorliegenden Fall beträgt diese Wartezeitspanne 120 ms. Dies bedeutet, dass im vorliegenden Beispiel (mit der Bildaufnahmerate von 40 Hz) o + 3 = 14 gilt. Das aus der Bildabfolge 19 als Kontrollbild herausgelesene Bild 19' hat in diesem Beispiel den Index i+p = i+14. Während der Belichtungszeit des Bildes 19' mit Index i+p, dem Kontrollbild, wird der Laserstrahl 11, welcher zu diesem Zeitpunkt als Ziellaser eingestellt ist, durch die Laserlenkvorrichtung 2 aus dem Sichtfeld des Sensor und somit insbesondere von der bestrahlten Stelle im Augenhintergrund weggelenkt, so dass die Bildinformationen dieser Stelle nicht durch den Ziellaserstrahl überdeckt werden. Ferner wird während der Aufnahme des Kontrollbildes das Auge mit automatisch sichtbarem Licht beleuchtet, so dass auf dem Kontrollbild eine durch die Laserbestrahlung hervorgerufene des Gewebes des Augenhintergrunds 16 an der bestrahlten Stelle als eine Bleichung sichtbar ist.

Die Steuereinheit 8 liest außerdem eine vorgegebene Zeitspanne vor der Laserbestrahlung ein weiteres als Referenzbild dienenden Kontrollbild aus der Kamera aus. Zu diesem Zweck weist die Steuereinheit 8 einen als Ringpuffer dienenden Speicher auf, der kontinuierlich mit Bildern 19' gefüllt wird, welche zeitlich mindestens bis zur obengenannten Zeitspanne zurückreichen. Zum Zeitpunkt des Auslösens der Laserbestrahlung wird ein Bild 19' mit einem zur Zeitspanne gehörigen Index aus dem Ringpuffer als Referenzbild ausgelesen. In diesem Beispiel beträgt diese Zeitspanne 160 ms , so dass das Referenzbild den Index i - m = i - 4 trägt. Während der Belichtungszeit dieses Bildes 19' wird der Augenhintergrund ebenfalls mit sichtbarem Licht beleuchtet, um das Referenzbild mit dem Kontrollbild vergleichen und somit physiologische Wirkungen der Laserbestrahlung auf dem Augenhintergrund beurteilen zu können.

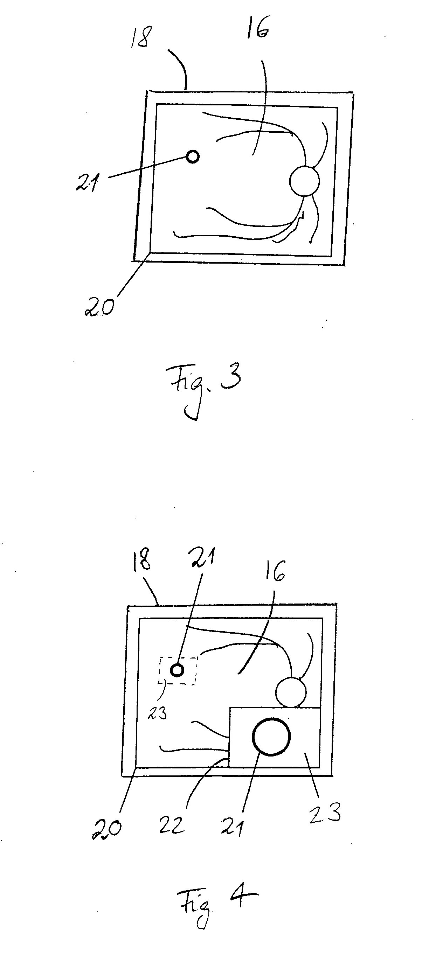

In Figur 3 ist eine schematische Darstellung eines beispielhaften Kontrollbilds 20, welches beispielsweise in gemäß dem anhand Figur 2 beschriebenen Verfahren aufgenommen wurde und auf einem Bildschirm 18 eines Ophthalmoskops hier vorgeschlagener Art, beispielsweise des anhand Figur 1 beschriebenen Ausführungsbeispiel 1, dargestellt wird. Dieses Kontrollbild wurde unter Beleuchtung mit sichtbarem Licht aufgenommen, so dass es Farbinformationen des Augenhintergrundes 16 beinhaltet. Insbesondere ist eine Läsion 21 als Bleichung zu erkennen, welche durch eine Laserbestrahlung des Augenhintergrundes 16 hervorgerufen wurde.

Das Kontrollbild 20 kann entweder in einer rein diagnostischen Kontrolluntersuchung des Auges 4 nach einem oben beschriebenen Verfahren erzeugt worden sein, wobei die Läsion 21 beispielsweise während einer bereits abgeschlossenen Behandlungssitzung hervorgerufen worden ist. Das in Figur 3 gezeigte Kontrollbild 20 kann aber auch während einer Laserbehandlung erzeugt werden. Dann wird das Kontrollbild 20 vorzugsweise unmittelbar nach seiner Erzeugung auf dem Bildschirm 18 angezeigt. Nach Betätigung eines Kontrollelements 9 des Ophthalmoskops oder später nach Ablauf einer Kontrollzeit, welche in diesem Beispiel 5 Sekunden andauert, wird dieses Kontrollbild 20 wieder ausgeblendet. Vor und nach dem Kontrollbild 20 wird auf dem Bildschirm 16 ein kontinuierliches unter infraroter Beleuchtung erzeugtes Echtzeitbild des Augenhintergrundes 16 gezeigt.

In Figur 4 zeigt wiederum schematisch ein Kontrollbild 20, welches sich von dem anhand Figur 3 beschriebenen Kontrollbild nur durch einen zusätzlich eingeblendeten Bildausschnitt 22 unterscheidet, welcher das Kontrollbild 20 teilweise überlagert. Zur Erzeugung dieses Bildausschnitts 22 wählt die Bildverarbeitungseinheit 10 einen Bildbereich 23 des Kontrollbildes aus, welcher die Läsion 21 umfasst, vergrößern diesen Bildbereich 23 und überlagert das Kontrollbild teilweise mit dem Bildbereich 23.

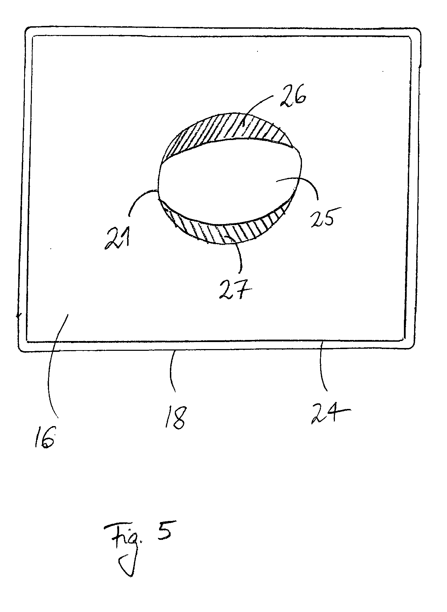

In Figur 5 ist ein Differenzwertbild 24 hier vorgeschlagener Art schematisch dargestellt, welches beispielsweise aus einem Kontrollbild und einem Referenzbild erzeugt wurde, welche ihrerseits gemäß einem der anhand Figur 2 beschriebenen Verfahren aufgenommen wurden. Zur Erzeugung des Differenzwertbildes 24, beispielsweise mittels des anhand Figur 1 beschriebenen Ausführungsbeispiel 1 der Erfindung, werden das Referenzbild und das Kontrollbild mittels einer merkmalbasierten Bildregistration miteinander verknüpft. Anschließend werden Differenzwerte zwischen Pixelwerten des Referenzbildes und des Kontrollbildes berechnet. Ein aus diesen Differenzwerten entstandenes Zwischenbild (hier nicht dargestellt) wird anhand von drei vorgegebenen Wertebereichen in Bildsegmente segmentiert, welche anhand der jeweiligen Wertebereiche einer ersten, einer zweiten und einer dritten Segmentklasse zugeordnet werden. Dabei bedeutet ein Bildsegment der ersten Segmentklasse einen Behandlungserfolg innerhalb dieses Bildsegments, ein Bildsegment der zweiten Segmentklasse eine notwendige Behandlungswiederholung innerhalb dieses Bildsegments und ein Bildsegment der dritten Segmentklasse eine notwendige Reduktion einer Laserintensität innerhalb dieses Bildsegments. Das Differenzwertbild wird schließlich in einer schwarzweißen Darstellung oder einer Falschfarbendarstellung auf einem Bildschirm 18 der Beobachtungsvorrichtung 17 abgebildet.

Wird also beispielsweise eine Behandlung des Augenhintergrundes 16 mittels eines anhand Figur 1 beschriebenen Ophthalmoskops durchgeführt, wird der Ziellaserstrahl 11 auf einen zu behandelnden Bereich des Augenhintergrundes 16 mittels der Laserlenkungsvorrichtung 3 positioniert. Dabei werden der Augenhintergrund unter infraroter Beleuchtung des Auges 4 sowie der Ziellaserstrahl als Echtzeitbild auf dem Bildschirm 18 der Beobachtungsvorrichtung 17 beobachtet. Anschließend wird eine Bestrahlung mit dem Therapielaserstrahl 11 durch die Steuereinheit 8 ausgelöst. Zu definierten Zeitpunkten vor und nach der Bestrahlung des Auges mit dem Therapielaserstrahl 11 wird das Auge 4 jeweils kurzzeitig mit einem Lichtimpuls von sichtbarem Licht beleuchtet. Außerdem werden die während dieser beiden Lichtimpulse durch die Kamera 6 erfassten Kontrollbilder aus der Kamera 6 ausgelesen, wie anhand Figur 2 beschrieben wurde. Anschließend wird aus den beiden Kontrollbildern mittels der entsprechend eingerichteten Bildverarbeitungseinheit 10 das oben beschriebene Differenzwertbild 5 erstellt und auf dem Bildschirm 18 der Beobachtungseinheit abgebildet.

Im vorliegenden Beispiel weist das gezeigte Differenzbild 24 drei Bildsegmente 25, 26, 27 auf, wobei das erste Bildsegment 25 der ersten Segmentklasse, das zweite Bildsegment 26 der zweiten Segmentklasse und das dritte Bildsegment 27 der dritten Segmentklasse angehört. Diesem beispielhaften Differenzwertbild entnimmt ein behandelnder Arzt über die Läsion 21 die Information, dass der zu behandelnde Bereich des Augenhintergrundes 16 innerhalb des Bildsegment 25 erfolgreich bestrahlt worden ist, während innerhalb des Bildsegments 26 die Bestrahlung nicht ausreichend war (und möglicherweise wiederholt werden sollte) und dass in dem dritten Bildsegment die Laserintensität zu hoch gewählt war.

Nach einer derartigen Interpretation des Differenzwertbilds 24 wird wieder das Echtzeitbild des Augenhintergrunds unter infraroter Beleuchtung angezeigt, wobei eine Umschaltung zum Echtzeitbild entweder durch den Arzt mittels der Eingabeschnittstelle 9 oder automatisch nach einer vorgegebenen Kontrollzeit durchgeführt, welche im vorliegenden Beispiel 5 Sekunden beträgt.

In einer alternativen Ausführungsform des zuletzt beschriebenen Verfahrens wird anstelle des Differenzwertbildes 4 das anhand Figur 3 oder Figur 4 beschriebene Kontrollbild 18 angezeigt. In einer weiteren Ausführungsform des Verfahrens wird das Kontrollbild 18 oder das Differenzwertbild 24 erst nach einer vorgegebenen Anzahl von durchgeführten Laserbestrahlungen des Auges 4 durchgeführt für eine Beschleunigung des Verfahrens. In einem entsprechenden Differenzwertbild 24 oder Kontrollbild 18 sind dann mehrere Läsionen gleichzeitig zu erkennen. In einer weiteren vorteilhaften Ausführungsform wählt der behandelnde Arzt über Eingabe entsprechender Kommandos über die Benutzerschnittstelle 9 zwischen den beschriebenen Darstellungen des Augenhintergrundes 16 durch die anhand Figuren 3 und 4 beschriebenen Kontrollbilder 18 oder durch das Differenzwertbild 24 aus.

Ein solches Behandlungsverfahren, insbesondere mittels eines Ophthalmoskops hier vorgeschlagener Art, hat den Vorteil, dass sich einzelne Arbeitsschritte einer Behandlung des Auges mittels einer Laserbestrahlung besonders gut automatisieren lassen, wie beispielsweise durch das beschriebene Ein- und Ausblenden von Kontrollbildern oder Differenzwertbildern unmittelbar nach Applikation einer Laserbestrahlung. Eine solche Automatisierung beschleunigt den Ablauf der Behandlung, insbesondere bei einer großer Anzahl einzeln durchzuführender Laserbestrahlungen in einer panretinalen Anwendung, und erleichtert gleichzeitig die Bedienung und Steuerung des Ophthalmoskops.

Die Anmeldung betrifft, inter alia, folgende Aspekte der Erfindung:
1. Ophthalmoskop (1) mit einer Laservorrichtung (2) zur Laserbestrahlung eines Auges (4), insbesondere zum Durchführen einer Photokoagulation auf einem Hintergrund (16) des Auges (4), umfassend eine Beleuchtungseinheit (5) zum Beleuchten des Auges (4) sowie eine Kamera (6) zum Erfassen eines Bildes (19') des Auges,
   dadurch gekennzeichnet, dass
   die Beleuchtungseinheit (5) eingerichtet ist zum Erzeugen von sichtbarem und infrarotem Licht, wobei das Ophthalmoskop (1) ferner eine Steuereinheit (8) umfasst, welche dazu eingerichtet ist die Beleuchtungseinheit (5) zum Erzeugen eines Lichtimpulses von sichtbarem Licht anzusteuern und ein während des Lichtimpulses von der Kamera (6) erfasstes Kontrollbild (18) des Auges (4) aus der Kamera (6) auszulesen.
2. Ophthalmoskop (1) aus Aspekt 1, dadurch gekennzeichnet, **dass** die Steuereinheit (8) eingerichtet ist nach einer vorgegebenen Wartezeitspanne nach einer Laserbestrahlung die Beleuchtungseinheit (5) zum Erzeugen des Lichtimpulses anzusteuern und das Kontrollbild (18) aus der Kamera auszulesen.
3. Ophthalmoskop (1) nach einem der vorangehenden Aspekte, dadurch gekennzeichnet, dass die Steuereinheit (8) eingerichtet ist nach einer vorgegebenen Wartezeitspanne nach Abschluss einer Serie einer vorgegebenen Anzahl einzelner Laserbestrahlungen die Beleuchtungseinheit (5) zum Erzeugen des Lichtimpulses anzusteuern und das Kontrollbild (18) aus der Kamera (6) auszulesen.
4. Ophthalmoskop (1) nach einem der vorangehenden Aspekte, dadurch gekennzeichnet, dass die Steuereinheit (8) eingerichtet ist eine vorgegebene Zeitspanne vor einer Laserbestrahlung die Beleuchtungseinheit (5) zum Erzeugen des Lichtimpulses anzusteuern und das Kontrollbild (18) als ein Referenzbild aus der Kamera (6) auszulesen.
5. Ophthalmoskop (1) nach einem der vorangehenden Aspekte, dadurch gekennzeichnet, dass die Steuereinheit (8) mit einer Eingabeschnittstelle (9) zum Eingeben eines Auslösesignals verbunden ist, wobei die Steuereinheit (8) dazu eingerichtet ist nach Eingabe des Auslösesignals die Beleuchtungseinheit (5) zum Erzeugen des Lichtimpulses anzusteuern und das Kontrollbild (18) aus der Kamera (6) auszulesen.
6. Ophthalmoskop (1) nach einem der vorangehenden Aspekte, dadurch gekennzeichnet, dass das Ophthalmoskop (1) eine mit der Kamera (6) verbundene Bildverarbeitungseinheit (10) beinhaltet, die dazu eingerichtet ist aus einem Paar von Kontrollbildern (18) ein Differenzwertbild (24) zu erzeugen.
7. Ophthalmoskop (1) nach einem der vorangehenden Aspekte, dadurch gekennzeichnet, dass die Bildverarbeitungseinheit (10) dazu eingerichtet ist, das Differenzwertbild (24) gemäß vorgegebener Wertebereiche für Differenzwerte des Differenzwertbildes (24) zu segmentieren.
8. Ophthalmoskop (1) nach einem der vorangehenden Aspekte, dadurch gekennzeichnet, dass die Bildverarbeitungseinheit (10) dazu eingerichtet ist das Differenzwertbild in Bildsegmente (25, 26, 27) einer ersten, einer zweiten und einer dritten Segmentklasse zu segmentieren, wobei ein Bildsegment (25) der ersten Segmentklasse einen Behandlungserfolg innerhalb dieses Bildsegments (25), ein Bildsegment (26) der zweiten Segmentklasse eine notwendige Behandlungswiederholung innerhalb dieses Bildsegments (27) und ein Bildsegment der dritten Segmentklasse eine notwendige Reduktion einer Laserintensität innerhalb dieses Bildsegments (27) signalisiert.
9. Ophthalmoskop (1) nach einem der vorangehenden Aspekte, dadurch gekennzeichnet, dass die Bildverarbeitungseinheit (10) dazu eingerichtet ist einen Bildausschnitt (22) eines Kontrollbildes (18) auszuwählen, zu vergrößern und das Kontrollbild (18) teilweise mit dem ausgewählten und vergrößerten Bildausschnitt (22) zu überlagern, wobei der ausgewählte und vergrößerte Bildausschnitt (22) einen behandelten Bereich des Auges beinhaltet.
10. Ophthalmoskop (1) nach einem der vorangehenden Aspekte, dadurch gekennzeichnet, dass die Laservorrichtung (2) eine Strahllenkungseinheit (3) beinhaltet zur Lenkung eines von der Laservorrichtung erzeugten Laserstrahls (11).
11. Ophthalmoskop (1) nach einem der vorangehenden Aspekte, dadurch gekennzeichnet, dass die Strahllenkungseinheit (3) eingerichtet ist einen Laserstrahl (11) der Laservorrichtung während des Lichtimpulses von sichtbarem Lichts aus einem Beobachtungsbereich heraus zu lenken.
12. Ophthalmoskop (1) nach einem der vorangehenden Aspekte, dadurch gekennzeichnet, dass die Beleuchtungseinheit (5) mindestens eine Leuchtdiode (5') umfasst.
13. Verfahren zum Erfassen eines Kontrollbilds eines Auges mit einem Ophthalmoskop (1), **dadurch** gekennzeichnet, **dass** es mit einem Ophthalmoskop (1) nach einem der Aspekte 1 bis 12 durchgeführt wird.
14. Verfahren zur Laserbehandlung eines Auges mit einem Ophthalmoskop (1), insbesondere zur Durchführung einer Photokoagulation eines Hintergrundes des (16) Auges (4),
   **dadurch gekennzeichnet, dass** mit einer Laservorrichtung (2) des Ophthalmoskops (1) eine Laserbestrahlung des Auges (4) durchgeführt wird und nach einer vorgegebenen Wartezeitspanne nach der Laserbestrahlung das Auge (4) mit einer Beleuchtungsvorrichtung (5) des Ophthalmoskops (1) mit einem Lichtimpuls von sichtbarem Licht beleuchtet wird und gleichzeitig mit einer Kamera (6) des Ophthalmoskops (1) ein Kontrollbild (18) des Auges (4) erfasst wird.
15. Verfahren aus Aspekt 14, dadurch gekennzeichnet, **dass** es mit einem Ophthalmoskop (1) nach einem der Aspekte 1 bis 12 durchgeführt wird.

### Bezugszeichenliste:

- 1: Ophthalmoskop
- 2: Laservorrichtung
- 3: Strahllenkungseinheit
- 4: Auge
- 5: Beleuchtungseinheit
- 5': Lichtquelle
- 6: Kamera
- 7: Sensor
- 8: Steuereinheit
- 9: Eingabeschnittstelle
- 10: Bildverarbeitungseinheit
- 11: Laserstrahl
- 12: Strahlteiler
- 13: Optisches System
- 14: Beleuchtungsstrahl
- 15: Beobachtungsstrahl
- 16: Augenhintergrund
- 17: Beobachtungsvorrichtung
- 18: Bildschirm
- 19: Bildfolge
- 19': Bild
- 20: Kontrollbild
- 21: Läsion
- 22: Bildausschnitt
- 23: Bildbereich
- 24: Differenzwertbild
- 25: Bildsegment der ersten Segmentklasse
- 26: Bildsegment der zweiten Segmentklasse
- 27: Bildsegment der dritten Segmentklasse

## Patentansprüche

1. Ophthalmoskop (1) mit einer Laservorrichtung (2) zur Laserbestrahlung eines Auges (4), insbesondere zum Durchführen einer Photokoagulation auf einem Hintergrund (16) des Auges (4), umfassend eine Beleuchtungseinheit (5) zum Beleuchten des Auges (4) sowie eine Kamera (6) zum Erfassen eines Bildes (19') des Auges, wobei die Beleuchtungseinheilt (5) eingerichtet ist zum Erzeugen von sichtbarem und infrarotem Licht, wobei das Ophthalmoskop (1) ferner eine Steuereinheit (8) umfasst, welche dazu eingerichtet ist, die Beleuchtungseinheit (5) zum Erzeugen eines Lichtimpulses von sichtbarem Licht anzusteuern und ein während des Lichtimpulses von der Kamera (6) erfasstes Kontrollbild (18) des Auges (4) aus der Kamera (6) auszulesen,
**dadurch gekennzeichnet, dass**
die Steuereinheit (8) eingerichtet ist nach einer vorgegebenen Wartezeitspanne mit einer Länge zwischen 1 ms und 5 s nach einer Laserbestrahlung die Beleuchtungseinheit (5) zum Erzeugen des Lichtimpulses anzusteuern und das Kontrollbild (18) aus der Kamera auszulesen.

2. Ophthalmoskop (1) nach Anspruch 1, **dadurch gekennzeichnet, dass** die Wartezeitspanne zwischen 40 ms und 1 s lang ist.

3. Ophthalmoskop (1) nach einem der vorangehenden Ansprüche, **dadurch gekennzeichnet, dass** die Steuereinheit (8) eingerichtet ist nach einer vorgegebenen Wartezeitspanne nach Abschluss einer Serie einer vorgegebenen Anzahl einzelner Laserbestrahlungen die Beleuchtungseinheit (5) zum Erzeugen des Lichtimpulses anzusteuern und das Kontrollbild (18) aus der Kamera (6) auszulesen.

4. Ophthalmoskop (1) nach einem der vorangehenden Ansprüche, **dadurch gekennzeichnet, dass** die Steuereinheit (8) eingerichtet ist eine vorgegebene Zeitspanne vor einer Laserbestrahlung die Beleuchtungseinheit (5) zum Erzeugen des Lichtimpulses anzusteuern und das Kontrollbild (18) als ein Referenzbild aus der Kamera (6) auszulesen.

5. Ophthalmoskop (1) nach einem der vorangehenden Ansprüche, **dadurch gekennzeichnet, dass** die Steuereinheit (8) mit einer Eingabeschnittstelle (9) zum Eingeben eines Auslösesignals verbunden ist, wobei die Steuereinheit (8) dazu eingerichtet ist nach Eingabe des Auslösesignals die Beleuchtungseinheit (5) zum Erzeugen des Lichtimpulses anzusteuern und das Kontrollbild (18) aus der Kamera (6) auszulesen.

6. Ophthalmoskop (1) nach einem der vorangehenden Ansprüche, **dadurch gekennzeichnet, dass** das Ophthalmoskop (1) eine mit der Kamera (6) verbundene Bildverarbeitungseinheit (10) beinhaltet, die dazu eingerichtet ist aus einem Paar von Kontrollbildern (18) ein Differenzwertbild (24) zu erzeugen.

7. Ophthalmoskop (1) nach einem der vorangehenden Ansprüche, **dadurch gekennzeichnet, dass** die Bildverarbeitungseinheit (10) dazu eingerichtet ist, das Differenzwertbild (24) gemäß vorgegebener Wertebereiche für Differenzwerte des Differenzwertbildes (24) zu segmentieren.

8. Ophthalmoskop (1) nach einem der vorangehenden Ansprüche, **dadurch gekennzeichnet, dass** die Bildverarbeitungseinheit (10) dazu eingerichtet ist das Differenzwertbild in Bildsegmente (25, 26, 27) einer ersten, einer zweiten und einer dritten Segmentklasse zu segmentieren, wobei ein Bildsegment (25) der ersten Segmentklasse einen Behandlungserfolg innerhalb dieses Bildsegments (25), ein Bildsegment (26) der zweiten Segmentklasse eine notwendige Behandlungswiederholung innerhalb dieses Bildsegments (26) und ein Bildsegment der dritten Segmentklasse eine notwendige Reduktion einer Laserintensität innerhalb dieses Bildsegments (27) signalisiert.

9. Ophthalmoskop (1) nach einem der vorangehenden Ansprüche, **dadurch gekennzeichnet, dass** die Bildverarbeitungseinheit (10) dazu eingerichtet ist einen Bildausschnitt (22) eines Kontrollbildes (18) auszuwählen, zu vergrößern und das Kontrollbild (18) teilweise mit dem ausgewählten und vergrößerten Bildausschnitt (22) zu überlagern, wobei der ausgewählte und vergrößerte Bildausschnitt (22) einen behandelten Bereich des Auges beinhaltet.

10. Ophthalmoskop (1) nach einem der vorangehenden Ansprüche, **dadurch gekennzeichnet, dass** die Laservorrichtung (2) eine Strahllenkungseinheit (3) beinhaltet zur Lenkung eines von der Laservorrichtung erzeugten Laserstrahls (11).

11. Ophthalmoskop (1) nach einem der vorangehenden Ansprüche, **dadurch gekennzeichnet, dass** die Strahllenkungseinheit (3) eingerichtet ist einen Laserstrahl (11) der Laservorrichtung während des Lichtimpulses von sichtbarem Lichts aus einem Beobachtungsbereich heraus zu lenken.

12. Ophthalmoskop (1) nach einem der vorangehenden Ansprüche, **dadurch gekennzeichnet, dass** die Beleuchtungseinheit (5) mindestens eine Leuchtdiode (5') umfasst.

## Claims

1. An ophthalmoscope (1) having a laser device (2) for laser irradiation of an eye (4) for carrying out a laser treatment of an eye (4), comprising an illuminator (5) for illuminating the eye (4) as well as a camera (6) for acquiring an image (19') of the eye, wherein said illuminator (5) is configured to generate visible and infrared light, the ophthalmoscope (1) further comprising a control unit (8) which is configured to trigger the illuminator (5) for generating a light pulse of visible light, and to read out from said camera (6) a control image (18) of the eye acquired by said camera (6) during the light pulse,
**characterized in that**
the control unit (8) is configured to trigger said illuminator (5) for generating a light pulse and to read out the control image (18) from the camera after a predetermined waiting time interval having a duration between 1 ms and 5s after a laser irradiation.

2. An ophthalmoscope (1) according to claim 1, **characterized in that** the wating time interval is between 40 ms and 1 s.

3. An ophthalmoscope (1) according to one of the preceding claims, **characterized in that** the control unit (8) is configured to trigger said illuminator (5) for generating the light pulse and to read out the control image (18) from the camera (6) after a predetermined waiting time interval after completion of a series of a predetermined number of individual laser irradiations.

4. An ophthalmoscope (1) according to one of the preceding claims, **characterized in that** the control unit (8) is configured to trigger the illuminator (5) for generating the light pulse and to read out the control image (18) as a reference image from the camera (6) a predetermined time interval before laser irradiation.

5. An ophthalmoscope (1) according to one of the preceding claims, **characterized in that** the control unit (8) is connected to an input interface (9) for inputting a trigger signal, wherein the control unit (8) is configured to trigger the illumi-nator (5) for generating the light pulse and to read out the control image (18) from the camera (6) after the input of the trigger signal.

6. An ophthalmoscope (1) according to one of the preceding claims, **characterized in that** the ophthalmoscope (1) comprises an image processing unit (10) connected to the camera (6) which is configured to generate a difference value image (24) from a pair of control images (18).

7. An ophthalmoscope (1) according to one of the preceding claims, **characterized in that** the image processing unit (10) is configured to segment the difference value image (24) according to predetermined value ranges for difference values of the difference value image (24).

8. An ophthalmoscope (1) according to one of the preceding claims, **characterized in that** the image processing unit (10) is configured to segment the difference value image into image segments (25, 26, 27) of a first, a second and a third segment class, wherein an image segment (25) of said first segment class signals a treatment success within this image segment (25), an image segment (26) of said second segment class signals a necessary repetition of the treatment within this image segment (26), and an image segment of the third segment class signals a necessary reduction of a laser intensity within this image segment (27).

9. An ophthalmoscope (1) according to one of the preceding claims, **characterized in that** the image processing unit (10) is configured to select and magnify an image detail (22) of a control image (18) and to partly superimpose the control image (18) with the selected and magnified image detail (22), wherein the selected and magnified image detail (22) comprises a treated region of the eye.

10. An ophthalmoscope (1) according to one of the preceding claims, **characterized in that** the laser device (2) comprises a beam guiding unit (3) for guiding a laser beam (11) generated by said laser device.

11. An ophthalmoscope (1) according to one of the preceding claims, **characterized in that** the beam guiding unit (3) is configured to guide out a laser beam (11) of the laser device from an observation range during the light pulse of visible light.

12. An ophthalmoscope (1) according to one of the preceding claims, **characterized in that** the illuminator (5) includes at least one light emitting diode (5').

## Revendications

1. Ophtalmoscope (1) avec un dispositif laser (2) pour l'irradiation par laser d'un oeil (4) en vue de l'exécution d'un traitement laser de l'oeil (4), comprenant une unité d'éclairage (5) pour l'éclairage de l'oeil (4) ainsi qu'une caméra (6) pour l'enregistrement d'une image (19') de l'oeil, dans lequel l'unité d'éclairage (5) est conçue pour produire de la lumière visible et infrarouge, l'ophtalmoscope (1) comprenant en outre une unité de commande (8) conçue pour commander l'unité d'éclairage (5) à produire une impulsion lumineuse de lumière visible, et pour lire une image de contrôle (18) de l'oeil (4) enregistrée par la caméra (6) pendant l'impulsion lumineuse, à partir de la caméra (6),
**caractérisé en ce que**
l'unité de commande (8) est conçue pour commander l'unité d'éclairage (5) à produire l'impulsion lumineuse et pour lire l'image de contrôle (18) à partir de la caméra, après un temps d'attente prédéfini d'une durée comprise entre 1 ms et 5 s suite à une irradiation par laser.

2. Ophtalmoscope (1) selon la revendication 1, **caractérisé en ce que** le temps d'attente dure entre 40 ms et 1 s.

3. Ophtalmoscope (1) selon l'une des revendications précédentes, **caractérisé en ce que** l'unité de commande (8) est conçue pour commander l'unité d'éclairage (5) à produire l'impulsion lumineuse et pour lire l'image de contrôle (18) à partir de la caméra (6), après un temps d'attente prédéfini après une série d'un nombre prédéfini d'irradiations par laser individuelles.

4. Ophtalmoscope (1) selon l'une des revendications précédentes, **caractérisé en ce que** l'unité de commande (8) est conçue pour commander l'unité d'éclairage (5) à produire l'impulsion lumineuse et pour lire l'image de contrôle (18) en tant qu'image de référence à partir de la caméra, par rapport à un laps de temps prédéfini avant une irradiation par laser.

5. Ophtalmoscope (1) selon l'une des revendications précédentes, **caractérisé en ce que** l'unité de commande (8) est reliée à une interface d'entrée (9) pour l'entrée d'un signal de déclenchement, l'unité de commande (8) étant conçue pour commander l'unité d'éclairage (5) à produire l'impulsion lumineuse et pour lire l'image de contrôle (18) à partir de la caméra (6) après l'entrée du signal de déclenchement.

6. Ophtalmoscope (1) selon l'une des revendications précédentes, **caractérisé en ce que** l'ophtalmoscope (1) contient une unité de traitement d'images (10) reliée à la caméra (6), laquelle est conçue pour produire une image de valeur différentielle (24) à partie d'une paire d'images de contrôle (18).

7. Ophtalmoscope (1) selon l'une des revendications précédentes, **caractérisé en ce que** l'unité de traitement d'images (10) est conçue pour segmenter l'image de valeur différentielle (24) selon des plages de valeur prédéfinies pour des valeurs différentielles de l'image de valeur différentielle (24).

8. Ophtalmoscope (1) selon l'une des revendications précédentes, **caractérisé en ce que** l'unité de traitement d'images (10) est conçue pour segmenter l'image de valeur différentielle en segments d'image (25, 26, 27) d'une première, d'une deuxième et d'une troisième classe de segments, où un segment d'image (25) de la première classe de segment indique une réussite de traitement dans ce segment d'image (25), un segment d'image (26) de la deuxième classe de segment indique la nécessité de répéter le traitement dans ce segment d'image (26), et un segment d'image de la troisième classe de segment indique la nécessité de réduire une intensité de laser dans ce segment d'image (27).

9. Ophtalmoscope (1) selon l'une des revendications précédentes, **caractérisé en ce que** l'unité de traitement d'images (10) est conçue pour sélectionner et agrandir un extrait d'image (22) d'une image de contrôle (18), et pour superposer partiellement l'image de contrôle (18) à l'extrait d'image (22) sélectionné et agrandi, l'extrait d'image (22) sélectionné et agrandi contenant une région traitée de l'oeil.

10. Ophtalmoscope (1) selon l'une des revendications précédentes, **caractérisé en ce que** le dispositif laser (2) contient une unité de guidage de faisceau (3) destinée à guider un faisceau laser (11) produit par le dispositif laser.

11. Ophtalmoscope (1) selon l'une des revendications précédentes, **caractérisé en ce que** l'unité de guidage de faisceau (3) est conçue pour guider un faisceau laser (11) du dispositif laser hors d'une région d'observation pendant l'impulsion lumineuse de lumière visible.

12. ophtalmoscope (1) selon l'une des revendications précédentes, **caractérisé en ce que** l'unité d'éclairage (5) comprend au moins une diode électroluminescente (5').
